# EUROPEAN PATENT SPECIFICATION

(11) **EP 3 340 951 B1**
(45) Date of publication and mention of the grant of the patent: **24.04.2024**
(21) Application number: 16839696.8
(22) Date of filing: 24.08.2016
(51) Int. Cl.: A61F 13/02, A61L 15/07, A61F 13/00, A61F 15/00

(54) **SURGICAL BANDAGE WITH STABILIZING ELEMENTS**
CHIRURGISCHER VERBAND MIT STABILISIERUNGSELEMENTEN
BANDAGE CHIRURGICAL DOTÉ D'ÉLÉMENTS DE STABILISATION

(30) Priority: 25.08.2015 SE 1500342
(43) Date of publication of application: 04.07.2018
(73) Proprietor: Swereco AB, 164 40 Kista (SE)
(72) Inventor: HEDENSTRÖM, John, SE-256 54 (SE); HAADEM, Knut, SE-254 33 Helsingborg (SE)
(74) Representative: Potter Clarkson
(86) International application number: PCT/SE2016/000042
(87) International publication number: WO 2017/034451

(56) References cited:
- EP-A1- 2 583 645
- WO-A1-98/31310
- WO-A1-2014/014842
- WO-A1-2015/110410
- US-A- 5 086 763
- US-A1- 2005 004 500
- US-A1- 2006 235 347
- US-A1- 2007 161 937
- US-A1- 2008 146 982
- US-A1- 2010 312 159
- US-A1- 2013 237 895
- US-A1- 2014 135 678

## Description

### Field of invention

The present invention pertains to a bandage for improved monitoring, healing and pain-reduction of a wound. The bandage also increases compliance for patients and for caring personal. The bandage is useful with most any wound being exposed to pressure, trauma and/or irritation. The bandage is especially useful with surgical incisions, preferably incisions upon abdominal and hip surgery as well as incisions upon dorsal surgery.

The above features are accomplished mainly by the wound being visible through the bandage, external pressure on the wound being redirected to areas around the wound not being compromised by surgery and possible exudate from the wound being absorbed within the bandage, as well as pressure from the bandage being exerted inwards against the wound edges thereby improving the cosmetic result.

### Background to the invention

The multifaceted problem of facilitating monitoring and healing wounds and pain reduction for a subject, while at the same time providing increased compliance for the subject and the caring personal is to date not satisfactorily solved.

The attempts that have been made to solve said problem have at most resulted in partial solutions. Examples of such unsatisfactory means are provided here below.

US 2207191754 (ONEX CORP) discloses a wound protecting and healing shield for a patient, having adhesive layer to secure conformable frame around wound, and access ports through the frame for insertion and removal of material from the enclosed space. This is in contrast to the present invention where access ports of the types of '754 are not provided.

US2010280428 (WIDGEROW A D) discloses a wound closure device, for shrinking and approximating opening a wound in multiple dimensions, having expandable dressing arranged such that absorption of wound exudate causes expansion of dressing. This is in contrast to the present invention where expansion of the dressing is not required.

DE3306383 (SCHELOWSKY M) discloses a cavity-forming dressing for treatment of wounds comprising circular pad, protective environmental mesh active substance cover and bandage strip. This is in contrast to the present invention where the dressing is not cavity forming and need not be circular.

US5462519 (CARVER J) discloses means for protecting bed sores on a person's body - using closed cell air bubble film with interior aperture registered with sore and opposed surfaces of film adhered respectively to person and bed. This is in contrast to the present invention where no part of the dressing is attached to a bed.

US2009088707 (MICHAEL A G) discloses device for promoting healing of skin cracks, has doughnut shaped foam pad, adhesive strip for forming reservoir in foam pad, and pressure-sensitive adhesive layer on other side of foam pad. This is in contrast to the present invention which is not for use with skin cracks.

GB225528 (TOKYO EIZAI LAB) discloses dressing comprising flexible plastics film and embossed non-woven fabric allowing application surface to be viewed through dressing. In contrast to the present invention the '528 dressing does not provide any dual function of reducing pain besides providing easy monitoring of the wound.

US201303030343 (ANTALEK M D) discloses a wound barrier pad to reduce pressure ulcers and bedsores in patient comprising housing having walls; surrounding internal wall with openings; and base plate with padding disposed in housing interior. In contrast to the present invention the `343 device lacks absorbing elements.

US5170781 (LOOMIS D L) discloses a bandage that protects a body area against impacts using fluid-filled bubble means being positioned centrally on the bandage and adapted to overlie the sensitive body area to protect it against impacts, to reduce the pain of impacts, and to promote healing. This is in contrast to the present invention where impact-reducing means are placed outside the body area to be protected.

US 2,280,506 (BETTS R T) discloses "a surgical dressing adapted to relieve pressure against sensitive or diseased parts of the body, such as corns, bunions or the like", see col.l, lines 2-5. This dressing comprises a cushioning element 12 confined within a recess 11, which prevents said cushioning element 12 from changing its shape. When the surgical dressing is applied on the patient said cushioning element 12 is placed on said sensitive or diseased parts of the body.

The correspondence in our invention to cushioning element 12 of `506 is stabilizing element/s 6, which is/are to be placed on "tissue not being compromised by surgery", see below.

US20030139697 (GILLMAN) discloses a wound closure dressing with controlled stretchability. The dressing comprises reinforcing members 12, which are the closest correspondence to our stabilizing element/s 6, which is/are to be placed on "tissue not being compromised by surgery", see below. In contrast thereto the reinforcing members 12 of `697 are to be placed on the wound.

US 8233313 (BELSON) discloses a surgical incision and closure apparatus with integrated force distribution. Our invention *inter alia* pertains to the closing of an incision, but not to the making of an incision.

US20100228287 (JEEKEL) relates to a device for adhering to the skin of a patient, suitable to make a skin incision there through, as well as to the use of such a device for allowing an incision or excision wound to be made through the said device, and for subsequent closing the wound. Our invention inter alia pertains to the closing of an incision, but not to the making of an incision. It is not possible to make any incision or excision wound through our claimed bandage.

US 2014/0135678 discloses devices, kits and methods for improved wound healing by applying and maintaining a predetermined strain in an elastic skin treatment device. WO 2014/014842 discloses a device making use of negative pressure for facilitating closure of wounds. WO 2015/110410 relates to a device making use of negative pressure for ameliorating closure of wounds. EP 2 583 645 Al discloses a skin lesion protector. WO 98/31310 discloses a non-contact controllable heated wound covering that includes a programmable active heater control.

No prior art document discloses a skin-contacting layer to be applied on the wound, which at the same time protects the same and stabilizes adjacent tissue not compromised by surgery.

In accordance with the invention, there is provided a bandage for use with a surgical incision on the skin of an animal or a human, characterized in that it comprises one or more stabilizing-and-pressure-absorbing-and-pressure-distributing element/s to be placed on the skin outside the incision, whereby said one or more element/s is/are provided with adhesive means; and wherein the bandage further comprises a skin-contacting layer, being attached to the skin-facing side of the one or more element/s, which is to be applied onto the incision, whereby the skin-contacting layer is large enough to cover some area outside the incision; and wherein the skin-contacting layer is transparent and is adhesive on its skin-facing side; and wherein the one or more elements is/are absorbents for water and blood components.

Design features and modes of operation of the claimed invention are provided in the below illustrative and non-limiting description and the accompanying figures.

### Legend of figures

The present figures are illustrative and non-limiting. White arrows denote compression. Black arrows denote expansion.

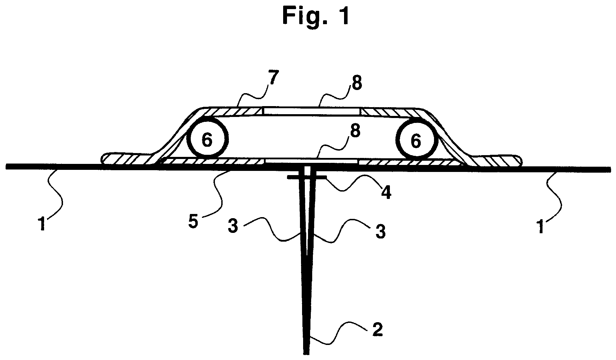

Figure 1 schematically shows in cross-section one embodiment of the inventive bandage as applied on the skin 1 of a human being or of an animal.

On the skin 1 is a wound 2 with wound edges 3 being held together with holding means 4, such as sutures, staples or tape. The wound is a surgical incision. The bandage comprises a skin-contacting layer 5, being at least partly perforated, e g with small openings, or provided with a network, intended for being placed on the wound 2. The bandage further comprises one or more stabilizing elements 6, which may be of varying form and cross section, a covering film 7, optional guiding means 8 on the skin-contacting layer 5 and/or in certain embodiments on the covering film 7 for facilitating orienting the bandage correctly on the wound 2. A release liner 9, not visible on the figures, may be placed on the skin-contacting layer 5 and/or the covering film 7 as a protection to be removed before the bandage being applied on the skin 1.

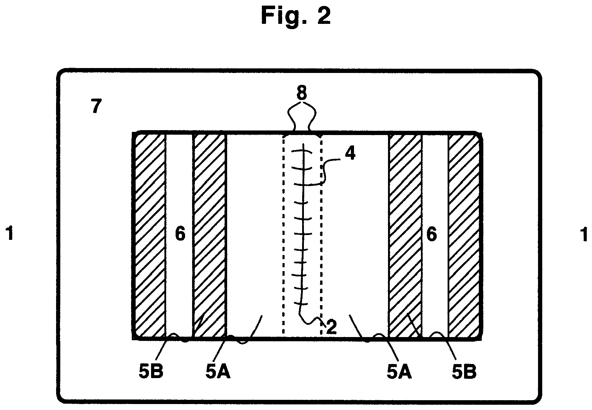

Figure 2 schematically shows from above an alternative embodiment of the present invention. Here 5A denotes a transparent, but not necessarily stretchable, part of the skin-contacting layer 5, while 5B denotes a stretchable, but not necessarily transparent, part of the skin-contacting layer 5.

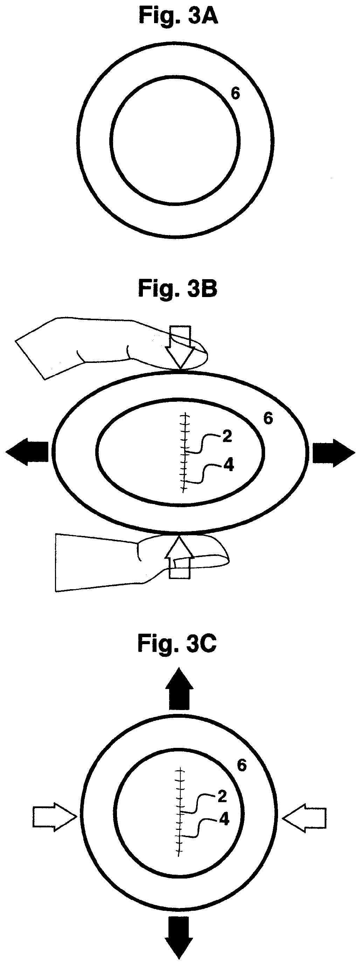

Figure 3 schematically shows from above a further alternative embodiment of the present invention. Here the wound 2, the skin-contacting layer 5 and the stabilizing element 6 are shown at different stages A, B and C. If needed the covering film 7, the optional guiding means 8 and the optional release liner 9 are adopted to suit this embodiment.

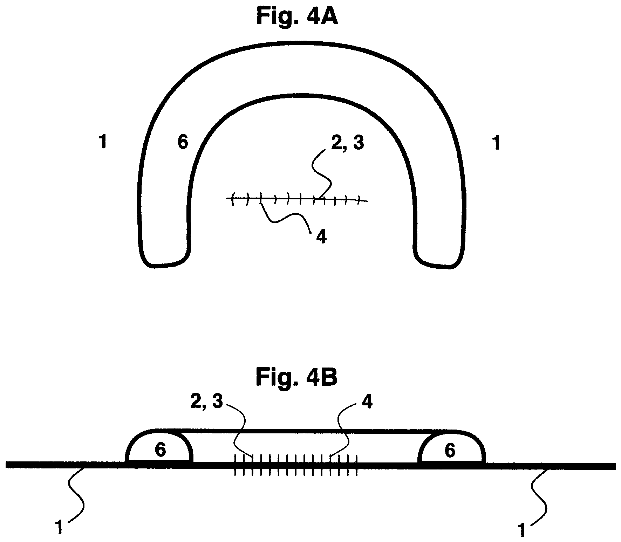

Figure 4 schematically shows from above and in cross-section a very simple surgical bandage not in accordance with the invention. This surgical bandage is essentially composed of one or more stabilizing elements 6, here also being pressure-absorbing-and-pressure-distributing, having one side for being attached on the skin 1 close to the wound 2, by way of fastening means 12, e g adhesive, micro-hooks and/or vacuum. An external pressure exercised on a dorsal surgical wound 2, e g the weight of a subject lying on his back, is redistributed from the wound 2 to an area outside the wound 2, said area not being compromised by surgery. This embodiment is useful mainly when there is no risk for exudate from the wound 2. The element/s 6 may have an open configuration, e g a horse-shoe form, or a closed configuration, e g a torus form. In this figure element 6 has a horse-shoe form.

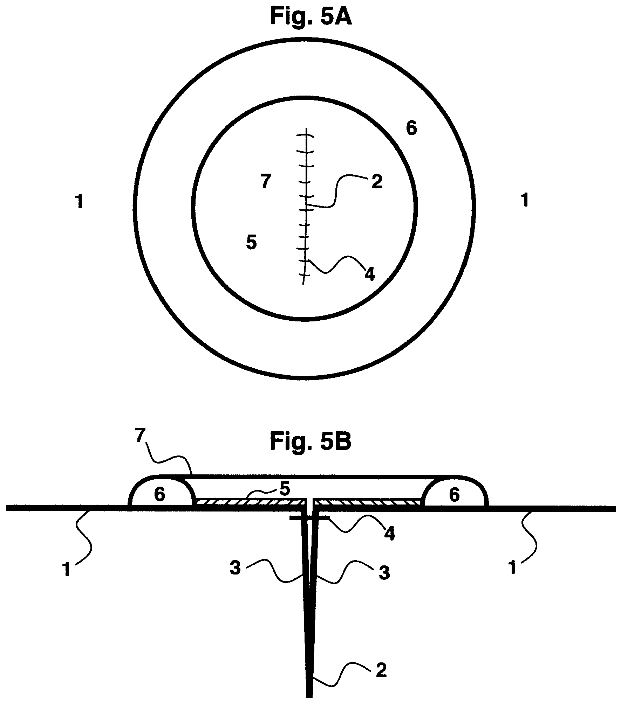

Figure 5 schematically shows from above a simple embodiment essentially being like the bandage of Figure 4 with the addition of a skin-contacting layer 5 and a covering film 7.

This embodiment is useful when there is risk for exudate from the wound 2. Element/s 6 need be a closed configuration, e g a torus or an elongated torus, in order to keep exudate within element/s 6.

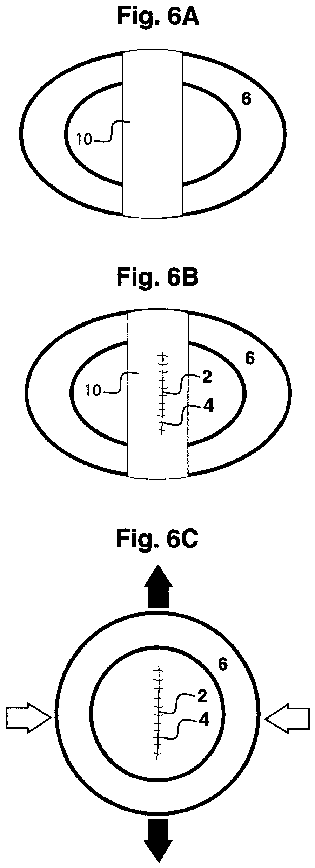

Figure 6A-6C schematically show from above a still further alternative embodiment of the present invention. What is here below said about element 6 is applicable mutatis mutandis on the claimed bandage as a whole. Figure 6A shows element 6 in a compressed state A before use. Element 6 is held in compressed state A by means of a holding device 10, e g an adhesive tape, a string or a clamp, here by way of example an adhesive tape. Figure 6B shows the bandage applied on the wound 2. Once the bandage is applied on the wound 2 the holding device 10 is removed.

As is shown in Figure 6C element/s 6 thereby strive/s to retake its/their non-compressed form. The actual physical change in element 6C might be very small. This is though not of great importance. Important is that the force exerted upon release of the holding device 10 is sufficient to keep the wound edges 3 firmly together.

It is though important that said force to keep the wound edges 3 firmly together is not so strong that possible exudate is stopped from exciting from the wound 2.

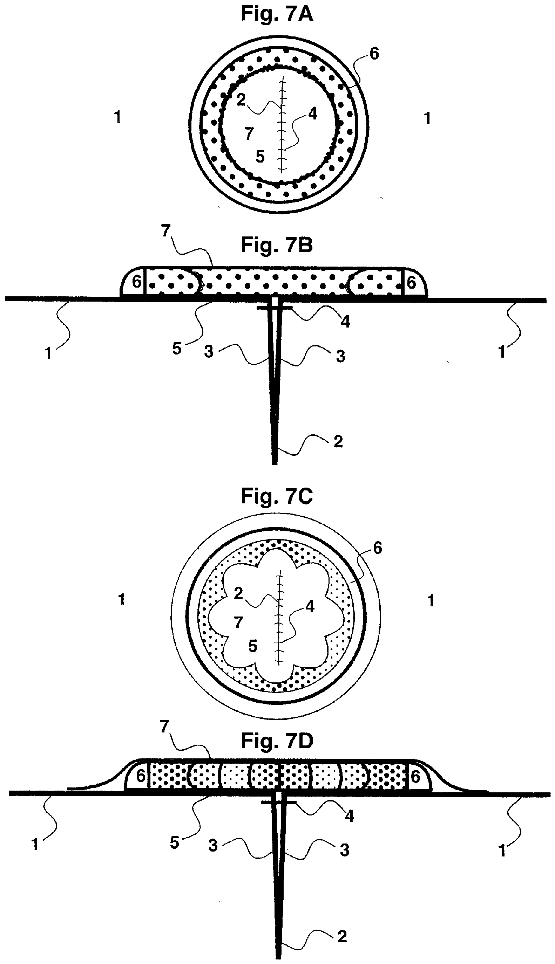

Figure 7 schematically shows from above and in cross-section a still further alternative embodiment of the present invention. Here the absorption area of the stabilizing element 6 is increased. By way of example the increase in absorption area is in this figure achieved by holes and by corrugation.

### Description of the invention

The invention is described by the below illustrative and non-limiting examples.

The present bandage solves the multifaceted problem of facilitating monitoring, treating, healing and pain-reducing wounds, which at least partly penetrates the skin of an animal or a human being, especially surgical incisions, preferably incisions upon abdominal, hip and dorsal surgery. The problem is solved by the present bandage, which also provides increased compliance for patients as well as for caring personal.

The present bandage e g provides the below effects and benefits
- provision of easy monitoring of the state of the wound, e g possible blood leakage,
- absorption within the bandage of possible exudate from the wound,
- enhanced healing of the wound due to the wound edges being pressed and adapted against one another by the bandage,
- increased cosmetic result due to the wound edges being pressed and adapted against one another by the bandage,
- reduced pain, e g when a patient with a surgical dorsal wound is lying down on his back the pressure is absorbed and redistributed by stabilizing elements that are placed at some distance from the wound on tissue not compromised by surgery,-creation of a sterile chamber above the wound, reducing the risk of the wound becoming infected.
- facilitated mounting of the bandage on a patient,
- facilitated mounting of an exchange bandage when needed.

### Example 1

Referring to Figure 1 the skin-contacting layer 5 is transparent, adhesive and permeable for liquids at least on an area covering the wound 2. The wound is a surgical incision. Permeability may be achieved by mechanical means, such as small holes, a network or by the material of the skin-contacting layer 5 being permeable as such. The bandage comprises a skin-contacting layer 5, being at least partly perforated, e g with small openings, or provided with a network. Other configurations and combinations thereof are also possible. Any suitable material in the art may be used, e g polymeric material. Adhesion of the skin-contacting layer 5 to the skin 1 may be achieved by chemical means, e g adhesives, and/or by mechanical means, e g a high-friction surface and micro-hooks.

In order to keep the wound edges 3 well together the skin-contacting layer 5 is preferably elastic and stretchable. The skin -contacting layer 5 should preferably be stretched when the bandage is applied on the skin 1.

In case there is no need to exert any pressure to keep the wound edges 3 well together a simpler embodiment without any skin-contacting layer 5 or with a skin-contacting layer 5 formed like a frame around the wound 2 may be used. In other words in this embodiment the skin-contacting layer 5 has a void central part, which at least covers the wound 2. Here the one or more stabilizing elements 6 may be attached to the covering film 7.

The skilled person may determine suitable width and breadth of the bandage. The skilled person may further see to it that also other parameters, e g compressibility and diameter of the element/s 6 and thickness of the covering film 7, be chosen so that the force applied on the bandage from e g the weight of a patient, will not cause the covering film 7 to stretch so much that it will touch the wound 2. Should that happen the intended protecting of the wound 2 from weight is not accomplished.

### Example 2

Elasticity of the skin-contacting layer 5 may be achieved by the material of the layer being elastic or stretchable as such. Referring to Figure 2 the skin-contacting layer 5 may alternatively be transparent and permeable only on the part covering the wound 2 and optionally a little more. The remaining part of the skin-contacting layer 5 is elastic or stretchable, but need be neither transparent nor permeable. One advantage in using two, or possibly more, materials is that you get a wider choice of stretchable materials. The stabilizing element/s 6 may preferably absorb liquid, such as water, blood and blood components. The stabilizing element/s 6 serve/s a dual function. They may absorb liquid leaking from the wound 2. They may also transfer the pressure exercised on the wound 2, e g when a person with a dorsal wound is lying on his back, to an area of the skin 1 being outside the wound 2. This transfer of pressure increases perceived compliance and reduces recovery time.

Once the skin-contacting layer 5 is applied on the wound 2 the covering film 7 is stretched and fastened on the skin 1. Thereby the stabilizing element/s 6 is/are pressed downwardly-inwardly so that the wound edges 3 are pressed against one another.

The covering film 7 is non-permeable for water, blood products and toxic elements. It preferably has a low friction coefficient on its non-skin-facing side to facilitate the patient's moving or sliding on the blanket in a bed.

The skin-contacting layer 5 and/or the covering film 7 may be provided with guiding means 8 for assisting in placing the bandage correctly on the wound 2. Said guiding means 8 may e g be dotted lines on the skin-contacting layer 5 and/or on the covering film 7.

If the adherence between the skin-contacting layer 5 and the skin 1 is satisfactory the skin-contacting layer 5 and/or the covering film 7 may not need extend outwardly beyond element 6. In many cases and as a precaution a stronger adherence between the skin-contacting layer 5 and the skin 1 is desired than what is thereby achievable. In such cases the skin-contacting layer 5 and/or the covering film 7 extend outwardly beyond element 6 in order to obtain a firmer grip of the bandage on the skin 1.

The bandage should preferably be kept in a sterile package until use. When applied on the wound 2 the bandage may keep a sterile compartment, namely the chamber 13 formed within the skin-contacting layer 5, the element/s 6 and the covering film 7. Having the wound 2 in direct contact with sterile chamber 13 facilitates and speeds up the healing of wound 2. Thereto also contributes that there is no pressure on the wound 2.

### Example 3

Figure 3 shows a useful embodiment for keeping the wound edges 3 together. The stabilizing element/s 6 is/are an elastic and closed circular loop. Other forms of the stabilizing element/s 6 are envisageable, such as oval, rectangular, hexagonal or octagonal. The stabilizing element/s 6 is elastic in itself or is rendered elastic by other means, e g by being placed within an elastic plastic ring. The stabilizing element/s 6 can have different, but preferably symmetrical, cross sections, such as square, hexagonal, octagonal, circular, and oval or combinations thereof.

The present embodiment is to be used mainly according to stages A, B and C of Figure 3. White arrows denote compression. Black arrows denote expansion.

Stage A: The stabilizing element/s 6 may e g form a circle when not being exercised. This stage A is the stage when the bandage is still in its package or simply prior to use.

Stage B: An optional release liner 9 is removed. The bandage is oriented over the wound 2 whereupon a pressure by hand or by using a tool, e g a pair of suitable tongs, is exerted on element/s 6 as shown with white arrows in Figure 3B.

Stage C: Once the bandage is stuck to the skin the slight pressure by hand or tool is released. Thereupon the elastic element/s 6 strive/s to get back to its/their circular form as shown with black arrows. Hereby the elastic skin-contacting layer 5 and the element/s 6 exert a pressure on the wound 2 pressing the wound edges 3 together. The actual change of the form of the elastic element/s 6 may be very small, while the exerted pressure on the wound 2 may still be substantial. The form of the cross section of element/s 6 may have an influence on how said pressure will exert. Element(s) 6 preferably form/s a closed figure being symmetrical along the wound 2.

The distance from the one or more elements 6 to the wound 2 may preferably be from about 0.5 cm to about 4 cm, more preferably about 1.5 cm.

### Example 4

Figure 4 schematically shows from above a very simple embodiment essentially composed of one or more stabilizing elements 6, here also being pressure-absorbing-and-pressure-distributing, having one side for being attached on the skin 1 at some distance from the wound 2, by way of e g an adhesive, micro-hooks or vacuum. An external pressure exercised on a dorsal surgical wound 2, e g the weight of a subject lying on his back, is redistributed from the wound 2 to an area around the wound 2 not being compromised by surgery. This embodiment is useful mainly when there is no risk for exudate from the wound 2. The element 6 may be an open configuration, e g a horse-shoe form, or a closed configuration, e g a torus.

### Example 5

Figure 5 schematically shows from above a simple embodiment essentially being like the embodiment of Figure 4 with the addition of a skin-contacting layer 5 and a covering film 7, This embodiment is useful when there is risk for exudate from the wound 2. Element 6 need be a closed configuration, e g a torus or an elongated torus.

### Example 6

Figures 6A-6C schematically show from above a still further alternative embodiment of the present invention.

The present embodiment is to be used mainly according to stages A, B and C of Figure 6. White arrows denote compression. Black arrows denote expansion.

What is here below said about element 6 is applicable *mutatis mutandis* on the whole claimed bandage as such. Figure 6A shows element 6 in a compressed state A before use. Element 6 is held in compressed state A by means of a holding device 10, e g an adhesive tape, a string or a clamp, here by way of example an adhesive tape. Figure 6B shows the bandage applied on the wound 2. Once the bandage is applied on the wound 2 the holding device 10 is removed. As is shown in Figure 6C element/s 6 thereby strive/s to retake its/their non-compressed form. The actual observable change between Figure 6B and Figure 6C might be very small. But, this is not important. The important issue is that the force exerted upon release of the holding device 10 is big enough to satisfactorily press the wound edges 3 against one another.

### Example 7

Figure 7 schematically shows still another useful alternative embodiment of the present invention. Here the absorption area of the stabilizing element/6 is/are increased by means of e g corrugation, folding, wrinkling, plying, flossing and/or provision of cavities. In Figure 7 the increase in absorption area is illustrated by holes and by corrugation.

In case the person wearing the bandage does not want the wound 2 to be permanently visible through the transparent part of the bandage said transparent part may be covered with a replaceable non-transparent cover 11, which may be removed when the wound 2 is inspected and replaced after such inspection. The replaceable non-transparent cover 11 may e g be a non-transparent adhesive tape. If a holding device 10 is used it may simultaneously serve the function of a replaceable non-transparent cover 11, e g by being a replaceable non-transparent adhesive tape, which may be a separate item or an integral part of the bandage.

In case there is no holding device 10 a replaceable and non-transparent cover 11 may be attached directly on the covering film 7. The holding device 10 and the replaceable non-transparent cover 11 may form one unitary item.

Material useful in the present invention, e g laminates, films, tapes, absorbents, pressure-distributing material and adhesives, should preferably be of pharmaceutically acceptable grade. There is an abundance of handbooks in this field being known by a person skilled in the art.

The present invention should preferably fulfill the requirements of wound care guidelines to support the prevention and treatment of surgical site infections (SSI) according to NICE (National Institute for Health & Clinical Excellence) in the UK, or corresponding guidelines, in order to minimize the risk for HCAl (healthcare-associated infection).

## Claims

1. A bandage for use with a surgical incision (2) on the skin (1) of an animal or a human, **characterized in that** it comprises one or more stabilizing-and-pressure-absorbing- and-pressure-distributing element/s (6) to be placed on the skin (1) outside the incision (2), whereby said one or more element/s (6) is/are provided with adhesive means; and wherein the bandage further comprises a skin-contacting layer (5), being attached to the skin-facing side of the one or more element/s (6), which is to be applied onto the incision (2), whereby the skin-contacting layer (5) is large enough to cover some area outside the incision (2); and wherein the skin-contacting layer (5) is transparent and is adhesive on its skin-facing side; and wherein the one or more elements (6) is/are absorbents for water and blood components.

2. A bandage according to claim 1, **characterized in that** the skin-contacting layer (5) has a void central part, which at least covers the incision (2).

3. A bandage according to claim 1 or claim 2, **characterized in that** it further comprises a covering film (7) affixed to the non-skin facing side of the one or more elements (6).

4. A bandage according to claim 3, **characterized in that** the covering film (7) extends beyond the skin-contacting layer (5) and the one or more elements (6) in order to add to the fastening of the bandage on the skin (1).

5. A bandage according to claim 3 or claim 4, **characterized in that** the covering film (7) is supplied with a replaceable non-transparent cover (11), which may be a separate item or an integral part of the bandage,.

6. A bandage according to any of claims 3 to 5, **characterized in that** the skin-contacting layer (5), the element/s (6) and the covering film (7) form a sterile chamber (13).

7. A bandage according to any of claims 3 to 6, **characterized in that** the covering film (7) is non-permeable for water and blood components.

8. A bandage according to any of claims 3 to 7, **characterized in that** it is provided with guiding means (8) for assisting in placing the bandage correctly on the incision (2), said guiding means (8), optionally comprising one or more markings on the skin-contacting layer (5) and/or on the covering film (7).

9. A bandage according to any of claims 3 to 8, **characterized in that** it comprises a protective release liner (9) on the skin-contacting layer (5) and/or on the covering film (7) to be removed before application of the bandage.

10. A bandage according to any of claims 1 to 6, **characterized in that** it comprises a protective release liner (9) on the skin-contacting layer (5) to be removed before application of the bandage.

11. A bandage according to any of claim 1 to 10, **characterized in that** the skin-contacting layer (5) is stretchable.

12. A bandage according to any of claims 1 to 11, **characterized in that** the skin-contacting layer (5) comprises a transparent part intended to at least cover the incision (2) and one or more stretchable parts being attached to said transparent part.

13. A bandage according to any of claims 1 to 12, **characterized in that** the skin-contacting layer (5) comprises a film, a network and/or strips or bridges.

14. A bandage according to any preceding claim, **characterized in that** it is provided with guiding means (8) for assisting in placing the bandage correctly on the incision (2), said guiding means (8) comprising one or more markings on the skin-contacting layer (5).

15. A bandage according to any preceding claim, **characterized in that** it is for use with abdominal, dorsal and/or hip surgical incisions; and optionally **characterized in that** the distance from the one or more stabilizing and pressure distributing elements (6) to the incision (2) is from about 0.5 cm to about 4 cm, optionally about 1.5 cm.

16. A bandage according to any preceding claim, **characterized in that** the cross-section of the one or more element(s) (6) is square, hexagonal, octagonal, circular and/or oval or any combination thereof.

17. A bandage according to any of claims 14 to 16, **characterized in that** the absorption area of the one or more element/s (6) is increased, by means of corrugation, folding, wrinkling, plying, flossing and/or provision of cavities.

## Patentansprüche

1. Verband zur Verwendung mit einer chirurgischen Schnittwunde (2) auf der Haut (1) eines Tiers oder eines Menschen, **dadurch gekennzeichnet, dass** er ein oder mehrere stabilisierende/s und druckabsorbierende/s und druckverteilende/s Element/e (6) umfasst, welche auf der Haut (1) außerhalb der Schnittwunde (2) platziert werden sollen, wobei das eine oder die mehreren Element/e (6) mit Haftmitteln versehen ist/sind; und wobei der Verband weiter eine hautkontaktierende Lage (5) umfasst, welche an der der Haut zugewandten Seite des einen Elements oder der mehreren Elemente (6) angebracht ist, welche auf die Schnittwunde (2) aufgelegt werden soll, wobei die hautkontaktierende Lage (5) groß genug ist, um etwas Fläche außerhalb der Schnittwunde (2) zu bedecken; und wobei die hautkontaktierende Lage (5) transparent ist und auf ihrer der Haut zugewandten Seite haftend ist; und wobei das eine oder die mehreren Element/e (6) Absorptionsmittel für Wasser und Blutkomponenten ist/sind.

2. Verband nach Anspruch 1, **dadurch gekennzeichnet, dass** die hautkontaktierende Lage (5) einen leeren zentralen Teil aufweist, welcher zumindest die Schnittwunde (2) bedeckt.

3. Verband nach Anspruch 1 oder Anspruch 2, **dadurch gekennzeichnet, dass** er weiter eine Deckschicht (7) umfasst, welche an der der Haut nicht zugewandten Seite des einen oder der mehreren Elemente (6) angebracht ist.

4. Verband nach Anspruch 3, **dadurch gekennzeichnet, dass** die Deckschicht (7) sich über die hautkontaktierende Lage (5) und das eine oder die mehreren Elemente (6) hinaus erstreckt, um die Befestigung des Verbands auf der Haut (1) zu unterstützen.

5. Verband nach Anspruch 3 oder Anspruch 4, **dadurch gekennzeichnet, dass** die Deckschicht (7) mit einer austauschbaren, nicht transparenten Bedeckung (11) versehen ist, welche ein getrennter Artikel oder ein integrierter Teil des Verbands sein kann.

6. Verband nach einem der Ansprüche 3 bis 5, **dadurch gekennzeichnet, dass** die hautkontaktierende Lage (5), das/die Element/e (6) und die Deckschicht (7) eine sterile Kammer (13) bilden.

7. Verband nach einem der Ansprüche 3 bis 6, **dadurch gekennzeichnet, dass** die Deckschicht (7) für Wasser und Blutkomponenten undurchlässig ist.

8. Verband nach einem der Ansprüche 3 bis 7, **dadurch gekennzeichnet, dass** er mit Führungsmitteln (8) versehen ist, um die korrekte Platzierung des Verbands auf der Schnittwunde (2) zu unterstützen, wobei die Führungsmittel (8) optional eine oder mehrere Markierungen auf der hautkontaktierenden Lage (5) und/oder der Deckschicht (7) umfassen.

9. Verband nach einem der Ansprüche 3 bis 8, **dadurch gekennzeichnet, dass** er eine Schutzfolie (9) auf der hautkontaktierenden Lage (5) und/oder auf der Deckschicht (7) umfasst, die vor Anwendung des Verbands entfernt werden muss.

10. Verband nach einem der Ansprüche 1 bis 6, **dadurch gekennzeichnet, dass** er eine Schutzfolie (9) auf der hautkontaktierenden Lage (5) umfasst, die vor Anwendung des Verbands entfernt werden muss.

11. Verband nach einem von Anspruch 1 bis 10, **dadurch gekennzeichnet, dass** die hautkontaktierende Lage (5) dehnbar ist.

12. Verband nach einem der Ansprüche 1 bis 11, **dadurch gekennzeichnet, dass** die hautkontaktierende Lage (5) einen transparenten Teil, welcher dazu vorgesehen ist, zumindest die Schnittwunde (2) zu bedecken, und einen oder mehrere dehnbare Teile umfasst, welche an dem transparenten Teil angebracht sind.

13. Verband nach einem der Ansprüche 1 bis 12, **dadurch gekennzeichnet, dass** die hautkontaktierende Lage (5) eine Folie, ein Netz und/oder Streifen oder Brücken umfasst.

14. Verband nach einem der vorstehenden Ansprüche, **dadurch gekennzeichnet, dass** er mit Führungsmitteln (8) versehen ist, um die korrekte Platzierung des Verbands auf der Schnittwunde (2) zu unterstützen, wobei die Führungsmittel (8) eine oder mehrere Markierungen auf der hautkontaktierenden Lage (5) umfassen.

15. Verband nach einem der vorstehenden Ansprüche, **dadurch gekennzeichnet, dass** er zur Verwendung bei chirurgischen abdominalen, dorsalen und/oder Hüftschnittwunden dient; und optional **dadurch gekennzeichnet, dass** der Abstand von dem einen oder den mehreren stabilisierenden und druckverteilenden Elementen (6) zu der Schnittwunde (2) von etwa 0,5 cm bis etwa 4 cm, optional etwa 1,5 cm beträgt.

16. Verband nach einem der vorstehenden Ansprüche, **dadurch gekennzeichnet, dass** der Querschnitt des einen Elements oder der mehreren Elemente (6) quadratisch, sechseckig, achteckig, rund und/oder oval oder jede Kombination davon ist.

17. Verband nach einem der Ansprüche 14 bis 16, **dadurch gekennzeichnet, dass** die Absorptionsfläche des einen Elements oder der mehreren Elemente (6) mithilfe von Rillen, Faltung, Knitterung, Schichtung, Flusen und/oder Bereitstellung von Hohlräumen erhöht ist.

## Revendications

1. Bandage destiné à être utilisé avec une incision chirurgicale (2) sur la peau (1) d'un animal ou d'un être humain, **caractérisé en ce qu'**il comprend un ou plusieurs éléments de stabilisation, d'absorption et de répartition de la pression (6) à placer sur la peau (1) à l'extérieur de l'incision (2), moyennant quoi le ou lesdits éléments (6) est pourvus de moyens adhésifs ; et dans lequel le bandage comprend en outre une couche en contact avec la peau (5), fixée au côté faisant face à la peau du ou des éléments (6), qui doit être appliquée sur l'incision (2), moyennant quoi la couche en contact avec la peau (5) est suffisamment grande pour couvrir une certaine zone à l'extérieur de l'incision (2) ; et dans lequel la couche en contact avec la peau (5) est transparente et est adhésive sur son côté faisant face à la peau ; et dans lequel le ou les éléments (6) sont des absorbants pour l'eau et les composants sanguins.

2. Bandage selon la revendication 1, **caractérisé en ce que** la couche en contact avec la peau (5) présente une partie centrale vide qui recouvre au moins l'incision (2) .

3. Bandage selon la revendication 1 ou la revendication 2, **caractérisé en ce qu'**il comprend en outre un film de recouvrement (7) fixé sur le côté non orienté vers la peau du ou des éléments (6).

4. Bandage selon la revendication 3, **caractérisé en ce que** le film de recouvrement (7) s'étend au-delà de la couche en contact avec la peau (5) et du ou des éléments (6) afin de renforcer la fixation du bandage sur la peau (1).

5. Bandage selon la revendication 3 ou la revendication 4, **caractérisé en ce que** le film de recouvrement (7) est muni d'un revêtement non transparent remplaçable (11), qui peut être un élément séparé ou une partie intégrante du bandage.

6. Bandage selon l'une quelconque des revendications 3 à 5, **caractérisé en ce que** la couche en contact avec la peau (5), le ou les éléments (6) et le film de recouvrement (7) forment une chambre stérile (13).

7. Bandage selon l'une quelconque des revendications 3 à 6, **caractérisé en ce que** le film de recouvrement (7) est imperméable à l'eau et aux composants sanguins.

8. Bandage selon l'une quelconque des revendications 3 à 7, **caractérisé en ce qu'**il est pourvu de moyens de guidage (8) pour aider à placer correctement le bandage sur l'incision (2), lesdits moyens de guidage (8) comprenant éventuellement un ou plusieurs marquages sur la couche en contact avec la peau (5) et/ou sur le film de recouvrement (7).

9. Bandage selon l'une quelconque des revendications 3 à 8, **caractérisé en ce qu'**il comprend une doublure protectrice détachable (9) sur la couche en contact avec la peau (5) et/ou sur le film de recouvrement (7) à retirer avant l'application du bandage.

10. Bandage selon l'une quelconque des revendications 1 à 6, **caractérisé en ce qu'**il comprend une doublure protectrice détachable (9) sur la couche en contact avec la peau (5) à retirer avant l'application du bandage.

11. Bandage selon l'une quelconque des revendications 1 à 10, **caractérisé en ce que** la couche en contact avec la peau (5) est étirable.

12. Bandage selon l'une quelconque des revendications 1 à 11, **caractérisé en ce que** la couche en contact avec la peau (5) comprend une partie transparente destinée à recouvrir au moins l'incision (2) et une ou plusieurs parties étirables qui sont fixées à ladite partie transparente.

13. Bandage selon l'une des revendications 1 à 12, **caractérisé en ce que** la couche en contact avec la peau (5) comprend un film, un réseau et/ou des bandes ou des ponts.

14. Bandage selon une quelconque revendication précédente, **caractérisé en ce qu'**il est pourvu de moyens de guidage (8) pour aider à placer correctement le bandage sur l'incision (2), lesdits moyens de guidage (8) comprenant un ou plusieurs marquages sur la couche en contact avec la peau(5).

15. Bandage selon une quelconque revendication précédente, **caractérisé en ce qu'**il est destiné à être utilisé avec des incisions chirurgicales abdominales, dorsales et/ou de la hanche ; et éventuellement **caractérisé en ce que** la distance entre le ou les éléments de stabilisation et de répartition de pression (6) et l'incision (2) est d'environ 0,5 cm à environ 4 cm, éventuellement d'environ 1,5 cm.

16. Bandage selon une quelconque revendication précédente, **caractérisé en ce que** la section transversale du ou des éléments (6) est carrée, hexagonale, octogonale, circulaire et/ou ovale ou toute combinaison de ceux-ci.

17. Bandage selon l'une quelconque des revendications 14 à 16, **caractérisé en ce que** la surface d'absorption du ou des éléments (6) est augmentée au moyen d'une ondulation, d'un pliage, d'un froissement, d'un retors, d'un fil dentaire et/ou de la création de cavités.
